# EUROPEAN PATENT APPLICATION

(11) **EP 0 913 160 A2**
(43) Date of publication of application: **06.05.1999**
(21) Application number: 98308935.0
(22) Date of filing: 30.10.1998
(51) Int. Cl.: A61K 49/00

(54) **Ligand-specific opening of a gated-porin channel in the outer membrane of living bacteria**

(30) Priority: 30.10.1997 US 64431 P
(71) Applicant: THE BOARD OF REGENTS OF THE UNIVERSITY OF OKLAHOMA, Norman, Oklahoma 73109 (US)
(72) Inventor: Klebba, Phillip F., Norman, Oklahoma 73019 (US); Newton, Salete M., Norman, Oklahoma 73019 (US)
(74) Representative: Baillie, Iain Cameron

(57) **Abstract**

The present invention relates in general to spectroscopic methods for direct observation of membrane dynamics in living cells and their application to drug discovery methods. More particularly, the present invention relates to methods for observing iron transport activity in the outer membrane of living bacteria and more specifically to a ligand-specific opening of a gated-porin channel in the outer membrane of a living bacteria.

## Description

### Brief Description of Invention

### 1. Field of the Invention

The present invention relates in general to spectroscopic methods for direct observation of membrane dynamics in living cells and their application to drug discovery methods. More particularly, the present invention relates to methods for observing iron transport activity in the outer membrane of living bacteria and more specifically to a ligand-specific opening of a gated-porin channel in the outer membrane of a living bacteria.

### 2. Brief Background Description of the Invention

Conceptual advances in science often derive from sudden experimental progress that permits the direct observation of previously inscrutable events. Whether accidental, as Pleming's discovery of the bacteriocidal effects of penicillin, or deliberate, as Perutz's use of the x-ray crystallography to characterize proteins and Watson and Crick's application of the method of DNA, direct observations of biological phenomena dramatically enriches the understanding of the natural world, creating unexpected technological, medical and economic benefits.

The present invention relates in general to the membrane biochemistry of pathogenic bacteria, which is described herein. The gram-negative bacteria manifests a dichotomous relationship to human biology and medicine. On the one hand, biochemists and molecular biologists have reaped a bountiful harvest of information from these organisms. With few exceptions, the basic pathways and mechanisms of life have emerged from the study of procaryotes. On the other hand, gram-negative bacteria, including for example *Escherichia coli, Vibrio cholerae, Salmonella typhi, Neisseria meningitidis* and *Yersinia pestis,* cause severe or fatal diarrhea, dysentery, septicemia, sexually-transmitted diseases, meningitis, and plague, and the world-wide mortality from these bacteria exceeds 2 million annually.

These organisms are surrounded by a selectively permeable membrane that permits the entry of essential nutrients and vitamins, but excludes noxious molecules like detergents, antibiotics, and toxins. Small molecules cross this membrane through open pores formed by proteins. Iron complexes, however, are too large to pass through the open pores and instead enter through larger channels that are normally closed. The necessity of iron in metabolism makes its acquisition a fundamental need of all living cells, including these disease causing organisms.

Because invasive bacteria must obtain iron in order to survive in the human body, the understanding of iron transport mechanisms is critical to efforts against disease. The invention described herein enables the observation of iron transport events as they happen, and provides a mechanism and methods of using the operation of these iron channels in the search for new drugs or as a triggering mechanism or biosensor for biomaterials. These iron uptake systems are valuable targets for therapeutic measures and the methods outlined herein provide direct methods for the identification of compounds that prevent their operation. Pathogenic bacteria can then be neutralized by either blocking the function of iron transport proteins, or using them as entry routes for antibiotics.

The past decade of research reveals the first details of membrane protein architecture. The methods outlined in the present invention signify an advance from a stage in which little was known about membrane protein structure, to the current situation in which x-ray crystallography has solved several prototypic membrane protein structures. Nevertheless, crystallographic depictions only provide a static view of proteins, leaving one of their most fascinating aspects, dynamic conformational changes during catalysis or transport, to theories and imagination.

Crystallography has rendered a detailed map of the redox centers within cytochrome oxidase, but this information does not clarify the exact path or mechanisms that electrons follow as they traverse through the protein toward final acceptance by molecular oxygen. Nor does the crystallographic structure reveal how electron movement through cytochrome oxidase leads to protein extrusion, the fundamental basis of energy generation in living cells.

Spectroscopic investigations of the proteins in vivo adds a new dimension to this area of research: a methodology capable of measuring the dynamic motions of proteins as they occur during a biochemical process. For the first time, the dynamic functioning of membrane transport proteins in bacteria has been observed. Where researchers formerly inferred that bacterial membrane transporters must recognize and bind the metal and then internalize it into the cell, the present invention, which utilizes genetic engineering and electron spin resonance (ESR), can directly observe this transporting action.

The technology characterized in the present invention thus addresses two important and intertwined issues: spectroscopy of living cells is an invaluable tool to basic biochemical research, and a major aim of the invention is its application to clinical medicine, for the identification of antibiotic compounds that will thwart pathogenic bacteria. Furthermore, the ability to use these iron transport systems as monitors for physiological changes within the body by using spectroscopic analysis of membrane protein function in living bacteria, pharmaceutical compounds that block iron channel operation can be sought after and evaluated.

These and other objects of the present invention will be apparent in light of the specification, drawings, and claims.

### BRIEF SUMMARY OF THE INVENTION

The present invention comprises a method for observing the dynamic functioning of membrane transport proteins in bacteria. Various techniques in genetic engineering and electron spin resonance (ESR) spectroscopy are used to directly observe this transporting action. Site-directed spectroscopy of living cells is a methodology suited to monitor many fundamental processes, especially the enigmatic and otherwise inaccessible events that transpire in biological membranes, which makes the invention widely relevant to research in molecular biology, biochemistry, physiology and medicine.

Ligand-gated membrane channels selectively facilitate the entry of iron into prokaryotic cells. The essential role of iron in metabolism makes its acquisition a determinant of bacterial pathogenesis and a target for therapeutic strategies. In Gram-negative bacteria, TonB-dependent outer membrane proteins form energized, gated pores that bind iron chelates (siderophores) and internalize them. Using electron spin resonance spectroscopy, the time-resolved operation of the Escherichia coli ferric enterobactin receptor, FepA, can be observed in vivo by monitoring the mobility of covalently-bound nitroxide spin labels. A ligand-binding surface loop of FepA, that normally closes its transmenbrane channel, exhibits energy-dependent structural changes during iron and toxin (colicin) transport. These changes are not merely associated with ligand binding, but occur during ligand uptake through the outer membrane bilayer. The results demonstrate by a physical method that gated-porin channels open and close during membrane transport in vivo.

### BRIEF DESCRIPTION OF THE SEVERAL VIEWS OF THE DRAWINGS

Fig. 1 depicts (A) an electro spin resonance (ESR) analysis of nitroxide labeled bacteria expressing FepAE280C gene, and (B) immunoassay results showing antigen-specific labeled protein using a western blot technique.

Fig. 2 depicts spectrographic results showing the effect of ferric enterobactin on the mobility of a nitroxide compound attached to the FepAE280C gene in live bacteria, observed in signal-averaged X-band spectra.

Fig. 3 depicts spectrographic results showing the effect of colicin B on the mobility of a nitroxide compound attached to the FepAE280C gene in live bacteria, observed in signal-averaged X-band spectra.

Fig. 4 depicts a statistical analysis of ferric enterobactin and colicin a induced variations in FepAE280C nitroxide motion.

Fig. 5 depicts spectrographic results showing the effect of TonB and energy dependence of conformational changes in FepAE280C.

### DETAILED DESCRIPTION OF THE INVENTION

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology or terminology employed herein is for purposed of description and should not be regarded as limiting.

One application of the present invention exists in the area of drug discovery: with ESR analysis of living bacteria, searches for and evaluation of new and existing pharmaceutical compounds for their ability to block iron channel operation can be readily accomplished. These types of drug discovery searches will have major clinical and economic impact by identifying novel antimicrobial compounds.

The use of ESR and fluorescence spectroscopy to monitor the *in vivo* biochemistry of *E. coli* proves several important concepts. The present invention shows that proteins in live bacteria may be labeled with physical probes on their external surfaces, that the labeled macromolecules function comparably to the analogous wild-type proteins, and that spectroscopic analysis of the labeled cells enables direct observations of biochemical processes and mechanisms in the bacterial outer membrane. The present invention can also be extended in two further directions: to pathogenic bacterial species, and to other compartments within the cell envelope.

*E. coli* may be highly virulent, as evidenced by the recent mortality attributed to the presence of this organism in contaminated meats, but it is usually perceived as a non-pathogenic commensal of the human gut. Conversely, many close and distant relatives of *E. coli* are unwavering or opportunistic pathogens that cause severe morbidity and mortality. The present invention also relates to the iron transport abilities of *Salmonella typhi*, *Pseudomonas aeruginosa*, and *Bordecella pertussis*, the causative agents of typhoid fever, burn infections, and whooping cough. The outer membrane receptors for ferric enterobactin of these bacteria, homologs of *E. coli* FepA, have been cloned and sequenced. These three organisms are difficult to treat with antibiotics, and infect distinctly different tissues: the intestinal tract, the skin, and the lungs. *In vivo* spectroscopy to the four bacterial species, delineates their mechanisms of iron transport and the ability of the present invention to study their antibiotic susceptibility.

Transport through biological membranes is selective and directional, providing a regulated influx of solutes required for metabolism and an efflux of molecules that function outside the cell. Ligand-gated membrane channels which exist in a variety of organisms and tissues, from bacteria to the human brain, form specific uptake pathways by binding solutes and moving them through transmembrane pores. In order to obtain iron, bacteria secrete small molecules called siderophores that bind Fe⁺⁺⁺ in the environment. In the outer membrane [OM] of Gram-negative bacteria, TonB-dependent gated porins transport iron in the form of ferric siderophores. These metal chelates are too big (750 daltons) to traverse the OM through the open channels of general porins, and channels large enough to accommodate them pose a threat to bacteria, because they would also allow the entry of detergents, antibiotics, and other noxious molecules. Gated porins have evolved that solve this nutritional dilemma by elaborating ligand-binding surface loops that close their large channels.

The activation of iron through ligand-gated porins is a determinant of bacterial pathogenesis, and the pores themselves are routes for the entry of toxins and antibiotics. The native siderophore of gram-negative bacteria is enterobactin; the *E. coli* outer membrane protein FepA recognizes and transports ferric enterobactin into the cell. A prototypical gated porin, *Escherichia coli* FepA, binds the siderophore ferric enterobactin in closed surface loops and transports it through an underlying transmembrane channel into the cell. The latter internalization reaction requires the input of energy and the participation of another protein, TonB.

FepA is one of a special class of proteins, called "TonB-dependent", that mediate the entry of iron and vitamins through the outer membrane of bacteria. Once in the periplasm, binding proteins selectively adsorb ferric complexes and mediate their movement to a group of proteins in the inner membrane, that transport iron into the cytoplasm. Hence the iron transport process occurs in three distinct phases, that involve the outer membrane, the periplasm, and the inner membrane. Each of the three transport stages embodies unique biochemical properties that have traditionally been poorly understood.

The energy for this transport process originates in the inner membrane, but passes to the outer membrane through TonB action, where it functions to catalyze conformational changes in FepA that deliver ferric enterobactin through the outer membrane into the periplasm. Thus the outer membrane transport stage alone involves specific ligand-receptor recognition events, protein-protein interactions that effect conformational changes, signal and energy transduction through three distinct cellular compartments, and the apparent opening of a normally closed membrane channel. The present invention relates to this mechanism of TonB action and virtually all high affinity iron acquisition systems of gram-negative bacteria require this cell envelope protein. The application of spectroscopic methods to TonB indicate its action in the uptake of ligands through the bacterial cell envelope. Furthermore, the universal dependence of procaryotic iron uptake mechanisms on TonB indicates that pharmaceuticals may be discovered or designed to generally inhibit iron acquisition in all Gram-negative pathogens.

Iron deprivation blocks bacterial proliferation. *In vivo* studies of iron channel function, as disclosed in the present invention, discloses a novel approach to the identification of bacteriocidal compounds spectroscopically identifying molecules that inhibit the transporting action of the FepA channel. A methodological paradigm for spectroscopic study of basic membrane biochemistry and its use for the identification of antimicrobial compounds, is hereby disclosed and claimed.

Because the TonB NH₂-terminus resides in the inner membrane and Tons promotes uptake through channels in the outer membrane, TonB-mediated transport reactions involve energy transduction between two distinct bilayers. By blocking the function of iron transport proteins, pathogenic bacteria can be neutralized.

The outer leaflet of the OM contacts the external environment, and various nutrients, noxious agents, and small molecules interact with OM proteins during the initial stages of transport. Because ferric enterobactin binds to the outside of a closed channel, through which it subsequently passes, conformational changes in PepA surface loops are a fundamental part of its transport mechanism. In order to confirm the conformational changes of FepA surface loops, nitroxide spin labels were reacted with a genetically engineered cysteine residue in a ligand-binding surface loop [PLS]. We then analyzed the live bacteria by electron spin resonance (ESR) spectroscopy during transport.

ESR detects the presence of unpaired electrons, and the introduction of a stable nitroxide free radical creates a molecular probe that conveys information about the structure and motional dynamics at its site of attachment. Site-directed spin labeling has generated extensive information about membrane proteins in vitro, including determinations of secondary and tertiary structure and characterization of conformational change. Previous ESR results with the purified mutant protein Glu²⁸⁰ → Cys²⁸⁰ (E280C), labeled with either S-(-oxyl-2,2,5,5-tetramethylpyrroline-3-methyl) methane thiosulfonate (MTSL) or 4-maleimido-tempo (MAL6) and studied in detergent or liposomes, suggested that FepA could be specifically modified and analyzed in vivo. Two findings confirmed this expectation: Bacteria harboring *pfepAE280C* were labeled 30- to 50-fold more than the *fepA* host strain KDF541 or KDF541 expressing the *fepA*^{*+*} allele that lacks the E280C mutation. Furthermore, in Western blots (protein immunoblots) of cell lysates with anti-MAL6 sera, the engineered Cys residue at FepA residue 280 was the major spin-labeled site in the bacteria.

The demonstration that the surface layer of wild-type bacteria is effectively devoid of modifiable cysteines is significant. The data indicates that appropriate target sites on the surface of an outer membrane protein may be genetically engineered such that they are receptive to Cys-specific covalent modification. Numerous paramagnetic and fluorescent Cys-specific labeling reagents are available as methanethiosulfonate (99% specific for Cys), maleimide (95%) and iodoacetamide (90%) derivatives, that react with proteins under gentle conditions (neutral or slightly basic buffers). Therefore, with correct structural insights and experimental design, virtually any outer membrane protein may be labeled, making its biochemical transport mechanisms accessible to experimental analysis.

*In vivo* spectroscopic experiments of this kind are not limited to *E. coli*. The cell envelopes of Gram-negative bacteria, including the pathogens of interest discussed above, manifest a common, trilaminar structure that includes an internal membrane surrounding the cytoplasm (the inner membrane), an external membrane that interacts with the environment (the outer membrane), and an aqueous compartment between the two bilayers (the periplasm) that contains many proteins, polysaccharides, and a structural polymer called peptidoglycan. The inner and outer membranes of Gram-negative bacteria harbor a multitude of clinically relevant biochemical systems, including the proteins and enzymes necessary for acquisition of nutrients for metabolism, for the biosynthesis and assembly of cell envelope components, for the generation of cellular energy, and for the excretion of noxious compounds like antibiotics. Through genetic engineering, clones of structural genes for most proteins of interest are already available on plasmids (e.g., the FepA proteins of the four organisms discussed above). With such plasmid-mediated systems we will design and introduce Cys substitutions in membrane proteins from the different bacteria, for attachment of ESR or fluorescent labels. the engineered proteins will be covalently modified and studied in *E. coli,* and, using shuttle vectors, in the bacteria from which they originated. Theoretically, this system for spectroscopic analysis of living cells is generally applicable to the study of membrane protein function in all procaryotes.

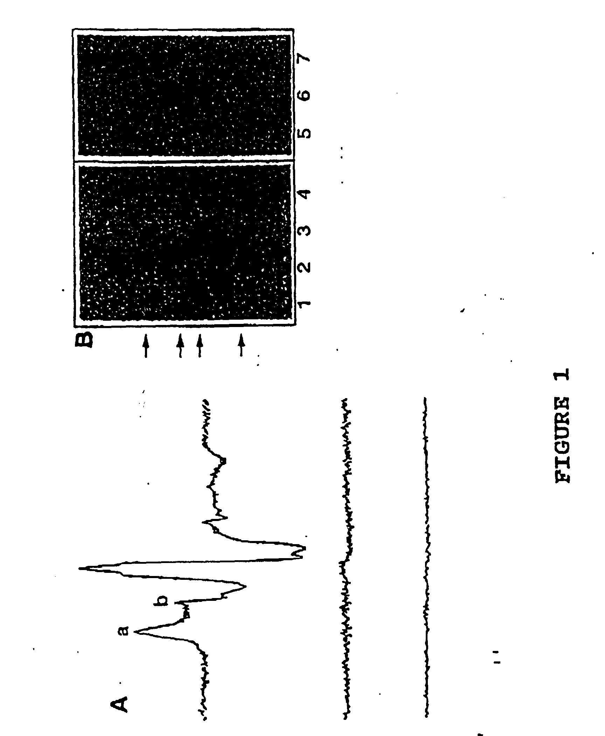

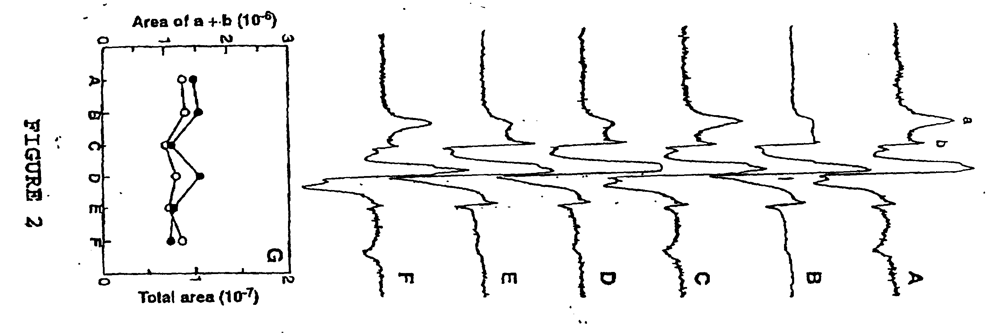

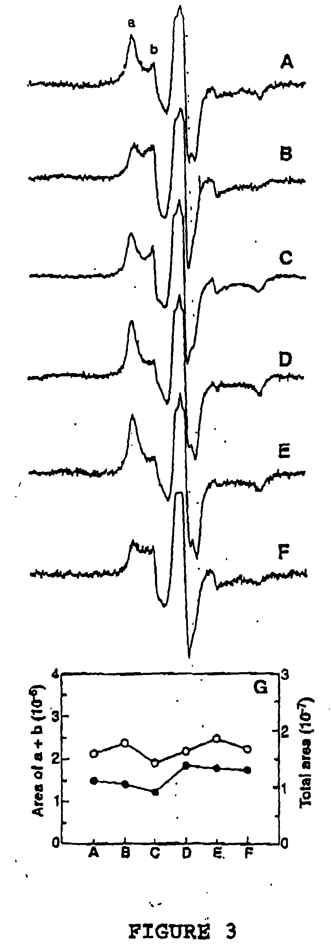

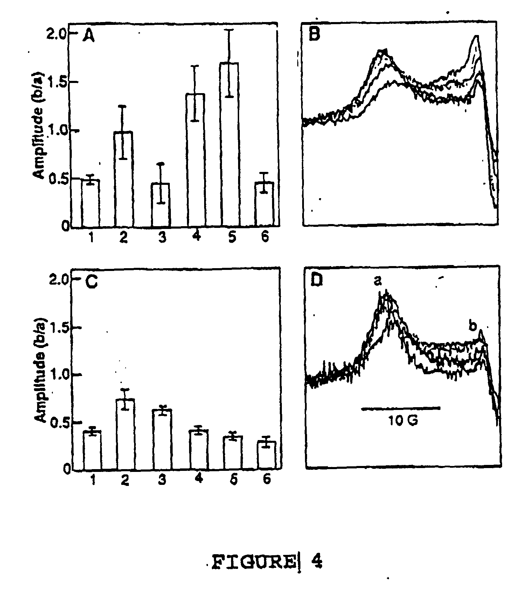

FIG. 1 shows nitroxide labeling of FepAE280C in vivo. Indicated at (A) are ESR analyses of MTSL-labeled bacteria expressing FepAE280C, KDF541(9) containing plTS449 of pFepAE280C was grown in LB medium and subcultured into T medium plus appropriate nutritional supplements at 1%. After 16 hours, bacteria were harvested and suspended in labeling buffer [50 mM Mops and 60 mM NaCl (PH7.5)]. Spin labels were added (to a final concentration of 20 µM MTSL or 10 µM MAL6) and incubated 2 hours at room temperature with shaking, and the cells were washed twice with reaction buffer containing 0.05% Tween-20 and once width reaction buffer alone. After the labeling reaction, the viability of the bacteria was confirmed by plating on LB agar. For ESR analysis, 10¹⁰ cells were suspended in 100 µl of 0.01 M NaHPO₄ (pH 6.9) containing 0.4% glucose. X-bend spectra of MTSL-labeled KDF541/pFepAE280C (top), KDF541/plTS449 (fepA⁺; center), or KDF541 (fepA; bottom) were recorded on a Bruker EMX spectrometer, operating at 9.8 GHz with a total scan range of 150 G and 100-kHz modulation frequency, at 4°C in a quartz flat cell. Peaks a and b show the position of strongly and weakly immobilized spin labels, respectively. Indicated at (B) are [¹²⁸I] -labeled protein A western blots with anti-MAL6 sera. MAL6 was coupled to bovine serum albumin (BSA) and ovalbumin (OVA). New Zealand White rabbits were immunized with BSA-MAL6 conjugates [100 µg/week for 1 month (29)], and polyclonal serum was collected in week 5 and assayed for antigen specificity by enzyme-linked immunosorbent assay (30) and Western blots against BSA, BSA-MAL6, OVA, and OVA-MAL6 (lanes 1 through 4, respectively). Lysates (5 x 10⁷ cells) of KDF541,KDF541/plTS449, and KDP541/pFepAE280C, previously labeled with MAL6, as described above, were reacted with the anti-MAL6 polyclonal serum (lanes 5 through 7, respectively). Arrows indicate the positions of (top to bottom) FepAE280C-MAL6, BSA-MAL6, BSA, and OVA-MAL6.

Although the in vivo ESR spectra of MTSL attached to residue 280 closely resembled its in vitro spectra, marked changes appeared during PepA-mediated transport in live bacteria. MTSL-labeled FepA showed a two-component spectrum in vitro, with the majority of its spins strongly immobilized and a small percentage (<1%) weakly immobilized. FepAE280C-MTSL showed similar spectra in vivo at 4°C (3% weakly immobilized), but an increase in sample temperature to 37°C in the presence of the siderophore shifted a significant proportion of the spin-label population to the weakly immobilized state.

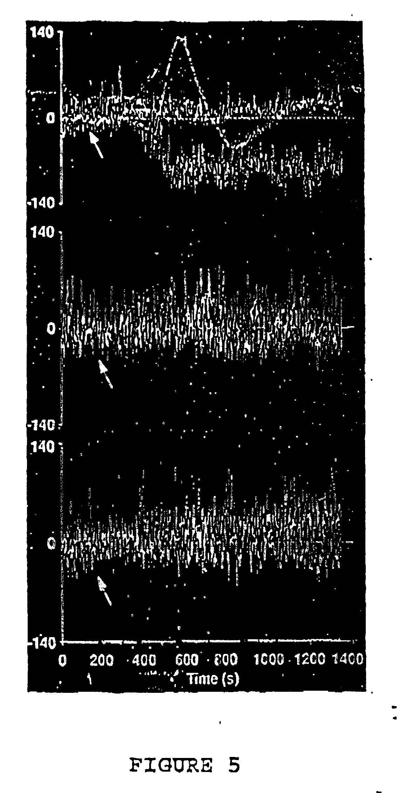

FIG. 2 shows the effect of ferric enterobactin on the mobility of MTSL attached to FepAE280C in live bacteria, observed in signal-averaged X-band spectra. KDF541/pFepAE280C was grown, spin-labeled, and prepared as described in FIG. 1. Signal-averaged X-band spectra (six field sweeps collected over an 8-minute period) of spin-labeled bacteria were recorded at 4°C as indicated at (A) and again after warming the flat cell to 37°C [indicated at (B) (31)]. A fresh aliquot of cells from the same labeling reaction was suspended in saturating ferric enterobactin (300 µM) at 4°C as indicated at (C), the sample was warned to 37°C, and spectra were recorded after 5 minutes, as indicated at (D), and 15 minutes, as indicated at (E) (midpoints of the signal-average measurements). The bacteria were then cooled again to 4°C for 30 minutes, and spectra were recorded, as indicated at (F). Each of the spectra was double-integrated, as indicated at (G), from 3406 to 3555 G to determine the total number of MTSL spins (O) and from 3443 to 3477 G to determine the spins derived from the strongly immobilized (a) and weakly immobilizod (b) peaks (●).

The effects of ferric enterobactin in FepA in vivo were distinct from those previously observed in vitro, and the binding of the siderophore caused a slight further immobilization of MTSL at E280C. The mobilization of spins in vivo was so conspicuous that we initially suspected chemical release of MTSL from FepA by ferric enterobactin. Other experiments refuted this possibility though. Recooling of the sample to 4°C reversed the effects, regenerating the properties and proportion of strongly immobilized spins attached to E280C (FIG. 2). Secondly, MAL6, which forms a chemically stable thioether bond with Cys, produced the same results with FepAE280C in vivo. Double integration of the X-band spectra, furthermore, showed that spin labels were neither destroyed nor lost during the experiment (FIG. 2).

The conversion of spins from strong to weak immobilization was comparable to changes in MTSL motion that accompany PepA denaturation, indicating that the siderophore stimulates relocation of PL5 away from globular protein structure into the aqueous milieu. The data shows that two distinct conformations of PL5 exist in vivo: a form that binds the siderophore, in which spin-label motion is restricted, and a form that arises after siderophore binding, in which spin-label motion is comparatively unrestricted. The appearance of the mobilized form coincided with the initiation of siderophore transport, indicating that ferric enterobactin triggered the conformational changes in FepA, either before or during its uptake.

Another TonB-dependent ligand that penetrates via FepA, colicin B, also altered MTSL mobility during its transport, but the effects were opposite to those engendered by the siderophore. The addition of saturating colicin B at 37°C progressively eliminated weakly immobilized spins, enhanced the strongly immobilized peak, and increased the separation between low- and high-field extreme (FIGS. 3 and 4).

FIG. 3 shows the effect of colicin B on the mobility of MTSL attached to FepAE280C in live bacteria, observed in signal-averaged X-band spectra. KDF541/pFepAE280C was grown, spin-labeled, and prepared as described in FIG. 1. Single-averaged X-band spectra (six field sweeps collected over an 8-minute period) of spin-labeled bacteria were recorded in the presence of saturating levels of colicin B (10 µM) at 4°C, as indicated at (A). The sample was warmed to 37°C and analyzed after 5 minutes, as indicated at (B), 15 minutes, as indicated at (C), and 75 minutes, as indicated at (D) (midpoints of signal average measurement before recooling to 4°C, as indicated at (E). The same strain was grown in minimal media and starved for glucose for 2 hours to deplete its energy stores, spin-labeled, and exposed so colicin B (10 µM) at 37°C for 30 minutes, as indicated at (F). The spectra were double-integrated, as indicated at (G), as described in FIG. 2.

FIG. 4 shows statistical analysis of ferric enterobactin- and colicin B-induced variations of FepAE280C-MTSL motion. KDF541/pFepAE280C was grown, spin-labeled, and prepared as described in FIG. 1 Multiple signal-averaged spectra were collected as in FIGS. 2 and 3 from several independent preparations of bacteria, and the mean and standard deviation of the ratio b/a was calculated at the indicated points after the addition of ferric enterobactin (A) (n=4) and colicin B (C) (n=6). The most sensitive measure of changes in spin-label mobility was the ratio of the emplitudes of b/a, shown and analyzed here. However, the effects of the siderophore and colicin were also analyzed by several other methods, including measurements of the absolute amplitudes, areas, separation of low- and high-field extrema (2T_{I}') and half-width at half-height (32) for peaks a and b. All these methods showed the same tendency of the siderophore to mobilize spin labels and the colicin to immobilize them. As indicated at (A), bars 1 through 6 correspond to samples A through F, respectively, of FIG. 2. As indicated at (C), bars 1, 2, 3, 5, and 6 correspond to panels A through E, respectively, of FIG. 3; bar 4 shows the effects of colicin after 30 minutes at 37°C. Superimposed spectra from single experiments are also shown in the region of the strongly and weakly immobilized peaks. As indicated at (B), spectra were collected in the absence of ferric enterobactin at 37°C (purple), in the presence of ferric enterobactin at 4°C (black). As indicated at (D), spectra were collected in the presence of colicin B at 4°C (purple), after increasing the temperature to 37°C for 5 minutes (blue), is minutes (green), and 75 minutes (red), and after recooling the sample to 4°C (black).

These changes did not result from colicin binding, because they did not take place during continual incubation of the cells at 4°C, even over the course of 1 Hour. However, when cells with bound colicin were warmed to 37°C, MTSL at residue 280 showed a progressive immobilization that became more severe over 30 min, ultimately converting virtually all Weakly immobilized spins to the strongly immobilized state (FIGS. 3 and 4).

As observed for the siderophore, these changes correlate with the transport phase of the colicin through the OM. It appears, therefore, that colicins traverse the OM bilayer through porin channels, and the progressive immobilization of MTSL by colicin B may reflect steric restriction of Spin-label motion as the colicin polypeptide passes through the FepA pore. The irreversibility of the immobilization during recooling supports the idea that the toxin polypeptide remains bound within FepA subsequent to OM transport. Both the kinetics and the nature of Colicin-induced effects differed from the actions of ferric enterobactin, which probably reflects the different steric constraints placed on the spin-labeled site during uptake of the two structurally diverse ligands.

Continuous monitoring of MTSL mobility (at 3472 G) over time showed that ferric enterobactin stimulated a burst of motion that peaked and receded over a 5-min period (FIG. 5).

FIG. 5 shows TonB and energy dependence of conformational changes in FepAE280C. As indicates at (top), *fepA, tonB*⁺ bacteria (KDF541) expressing FepAE280C were spin-labeled with MTSL and analyzed in the presence of glucose. The intensity of the weakly immobilized peak (b in FIG. 1) was monitored versus time by setting the spectrometer center magnetic field at the peak maximum (3472.3 G) and the sweep width to zero. Bacteria were incubated at 4°C without added ligands (blue tracing) and warmed to 37°C after 5 minutes (marked by an arrow). The experiment was repeated in the presence of ferric enterobactin, (300 µM; yellow) or colicin B (10 µM; red), as described in FIGS. 2 and 3. As indicated at (center) *fepA*, *tonB*^{*+*} bacteria (KDF541) expressing FepAE280C were analyzed, but were energy-depleted by glucose starvation described in FIG. 3 and subjected to the conditions described above. As indicated at (bottom), analysis of *fepA, tonB* bacteria (KDF570(9)] expressing FepA280C, subjected to the conditions described above.

This surge in motion accrued from the sum of individual, weakly immobilized MTSL spins in the bacterial sample. The changing mobility of the spin label in PLS reflects the opening and closing of the gated FepA channel during siderophore uptake. Kinetic analysis of the spin-label motion tracing, which showed two peaks of mobility approximately 10 and 20 min after the temperature shift to 37°C, revealed in both cases a sigmoidal rise to a maximum followed by an approximately symmetrical decay to a minium. The integrated areas beneath the two curves were the same, indicating that the changes in motion arose from an initial, relatively synchronous transport cycle and a second, less synchronous cycle in the same number of cells. The first surge in motion peaked in 140 seconds and decayed in 140 seconds; because ferric enterobactin was supplied in excess, the decrease in motion did not result from its depletion. Thus, the time-resolved fluctuations in PL5 were consistent with an active opening and closing of the channel during the ferric enterobactin transport reaction.

The relocation of spin labels attached to E280C, by both ligands, indicated the hallmarks of energy and TonB-dependence: Glucose deprivation, low temperatures, and use of tonB strain all prevented the effects. During ligand internalization , FepA PL5 changed to either mobilize (by decreasing their contact with other components of FepA structure) or immobilize [by increasing their contact with the FepA polypeptide or the protein (colicin B) that interacted with it] the attached spin labels.

Hence, FepA fluctuates between at least two conformations during ferric enterobactin transport, and colicin entry creates a different kind of conformational dynamic. The TonB and energy dependence of the observed structural changes confirms their physiological relevance and the ligand-specific oscillation of probes attached to FepA PL5 suggests how siderophores enter the normally closed pores, TonB triggers energy-dependent structural changes in the ligand-binding site that displace PL5 and release the bound siderophore into the FepA channel. These results constitute physical evidence for the in vivo operation of a ligand-gated channel. They demonstrate what has been widely hypothesized, that gated porins open and close during solute transport. The perception of OM porins as rigid molecules containing passive channels, derived from crystallographic studies in vitro, contrasts with this characterization of FepA as a dynamic entity in vivo.

Although the components and transport functions of the outer membrane are well defined, the periplasm and inner membrane are largely inaccessible to *in vivo* experimentation. The present invention's mutant bacteria with enhanced outer membrane permeability, which permit entry of spin and fluorescent labels into the periplasm, and mutant bacteria with deficiencies in periplasmic thiol metabolism (*dsbA/dsbB*) which permit modification of cysteine in the periplasm, allow for the study and characterization of these inaccessible membrane areas. The benefits of site-directed labeling in the periplasm are substantial, as the major protein components of the energy-generating machinery of bacteria, all of the cellular transport systems, and several antibiotic-resistance processes reside therein.

A second application of the present invention is that it identifies a molecule (protein) that opens and closes - a biological switch that operates on the molecular level. Although the protein itself was previously known, our invention demonstrates and describes methods of using its opening and closing action. An interesting aspect of this particular membrane protein is that it contains a pore or channel, through which charged molecules pass. This pore is, and could serve as, an electrical conduit that opens and closes in response to specific biological processes. Hence it has commercial application in the area of biosensor technology. For example, if incorporated on a microchip, the pore protein could serve as a sensor that conducts an electrical current in response to certain biological events. Application of this technology is appropriate to the fields of medicine (turning on or off prosthetic pumps or other devices) and biotechnology (interfacing living biological systems with computers).

Thus, it should be apparent that there has been provided in accordance with the present invention methods for observing iron transport activity in the outer membrane of living bacteria as well as methods for using the outer membrane iron transport activity in biomechanical and biotechnical applications, that fully satisfy the objectives and advantages set forth hereinabove. Although the invention has been described in conjunction with specific embodiments thereof, it is evident that many alternatives, modifications, and variations will be apparent to those skilled in the art. Accordingly, it is intended to embrace all such alternatives, modifications, and variations that fall within the spirit and broad scope of the appended claims.

## Claims

1. A method for observing in vivo membrane transport in bacteria, comprising the steps of:
- spin-labeling membrane transport sites to provide a spin-labeled membrane transport site; and
- measuring conformational changes of the spin-labeled membrane transport sites.

2. The method of claim 1, further comprising the steps of:
- associating a nitroxide compound to the spin-labeled membrane transport site to provide a spin-labeled membrane transport site associated with a nitroxide compound; and
- measuring conformational changes of the spin-labeled membrane transport site associated with a nitroxide compound.

3. A method for observing in vivo molecular motion during iron channel transport in living bacteria, comprising the steps of:
- modifying iron channel surface bacterial residues to provide a modified bacterial residue;
- labeling the modified bacterial residue to provide a labeled modified bacterial residue;
- reacting the labels modified bacterial residue with an iron-binding protein; and
- measuring conformational changes in the labeled modified bacterial residue and the iron-binding protein while the labeled modified bacterial residue reacts with the iron-binding protein.

4. The method of claim 3, further comprising the step of measuring the effect of temperature on the reaction between the labeled modified bacterial residue and the iron-binding protein.

5. A method for delivering therapeutic agents within the human body, comprising the steps of:
- providing a means for sensing iron transport activity in a bacteria; and
- providing a means for delivering a therapeutic agent through the iron transport activity in the bacteria.

6. A method for observing membrane transport, comprising the steps of:
- spin-labeling a site to provide a spin-labeled site; and
- measuring a change of the spin-labeled site.

7. A method for observing molecular motion, comprising the steps of:
- reacting a labeled modified surface residue with a protein, and
- measuring a change in the labeled modified surface residue and/or protein.

8. A method for delivering a therapeutic agent within a mammalian body, comprising the steps of:
- providing a means for sensing transport activity in a bacteria; and
- providing a means for delivering a therapeutic agent through the transport activity in the bacteria.
